# EUROPEAN PATENT SPECIFICATION

(11) **EP 3 752 597 B1**
(45) Date of publication and mention of the grant of the patent: **18.03.2026**
(21) Application number: 19703750.0
(22) Date of filing: 14.02.2019
(51) Int. Cl.: C12N 5/076, C12N 5/071, A01K 67/00, G01N 33/68

(54) **METHOD FOR OBTAINING A SPERMATOZOID CELL POPULATION WITH IMPROVED FITNESS**
VERFAHREN ZUR HERSTELLUNG EINER SPERMATOZOIDENZELLPOPULATION MIT VERBESSERTER FITNESS
PROCÉDÉ D'OBTENTION D'UNE POPULATION CELLULAIRE DE SPERMATOZOÏDES À APTITUDES AMÉLIORÉES

(30) Priority: 14.02.2018 EP 18382084
(43) Date of publication of application: 23.12.2020
(73) Proprietor: Universidad del País Vasco/Euskal Herriko Unibertsitatea, 48940 Leioa, Vizcaya (ES)
(72) Inventor: SUBIRÁN CIUDAD, Nerea, 48940 Leioa - Vizcaya (ES); IRAZUSTA ASTIAZARAN, Jon, 48940 Leioa - Vizcaya (ES); CASIS SAENZ, Luis, 48940 Leioa - Vizcaya (ES); URIZAR ARENAZA, Itziar, 48940 Leioa - Vizcaya (ES); MUÑOA HOYOS, Iraia, 48940 Leioa - Vizcaya (ES); GIANZO CITORES, Marta, 48940 Leioa - Vizcaya (ES)
(74) Representative: ABG Intellectual Property Law, S.L.
(86) International application number: PCT/EP2019/053615
(87) International publication number: WO 2019/158621

(56) References cited:
- TANAKA ATSUSHI ET AL: "Fourteen babies born after round spermatid injection into human oocytes", PROCEEDINGS OF THE NATIONAL ACADEMY OF SCIENCES (PNAS), vol. 112, no. 47, 2 November 2015 (2015-11-02), pages 14629 - 14634, XP093286728, ISSN: 0027-8424, DOI: 10.1073/pnas.1517466112
- "Emery and Rimoin's Principles and Practice of Medical Genetics and Genomics (Seventh Edition)", 1 January 2022, ELSEVIER, article KRAUSZ CSILLA ET AL: "Chapter 6 Genetics of Male Infertility", pages: 121 - 147, XP093286725
- GIANZO MARTA ET AL: "Angiotensin II type 2 receptor is expressed in human sperm cells and is involved in sperm motility", FERTILITY AND STERILITY, ELSEVIER SCIENCE INC, NEW YORK, NY, USA, vol. 105, no. 3, 23 November 2015 (2015-11-23), pages 608 - 616, XP029449902, ISSN: 0015-0282, DOI: 10.1016/J.FERTNSTERT.2015.11.004
- NEREA SUBIRÁN ET AL: "Control of APN/CD13 and NEP/CD10 on sperm motility", ASIAN JOURNAL OF ANDROLOGY, vol. 12, no. 6, 20 September 2010 (2010-09-20), US, pages 899 - 902, XP055484846, ISSN: 1008-682X, DOI: 10.1038/aja.2010.82
- JEFF WANG ET AL: "In vitro fertilization (IVF): a review of 3 decades of clinical innovation and technological advancement", THERAPEUTICS AND CLINICAL RISK MANAGEMENT, vol. 2, no. 4, 1 December 2006 (2006-12-01), NZ, pages 355 - 364, XP055485549, ISSN: 1176-6336, DOI: 10.2147/tcrm.2006.2.4.355

## Description

### Field of the Invention

The present invention relates to the field of assisted reproduction and, more in particular, to a method for obtaining a spermatozoid cell population enriched in CD10⁻ spermatozoids with improved fitness which are more effective in Assisted Reproduction Techniques (ART) thereby reducing the number of infertile couples undergoing ART that fail to achieve pregnancy. The invention also relates to a method for determining the fitness of a sperm population for fertilization based on the content of CD10⁻ spermatozoids. The invention can also be used in the field of veterinary for the selection and improvement of species.

### Background of the Invention

The current prevalence of infertility is estimated to be around 14% worldwide and couples that experience infertility problems is increasing each year. It has been estimated that over 186 million people worldwide have infertility problems during their reproductive lifetime and male factor represents the 50% of clinical infertility cases. The ART (Assisted Reproduction Techniques) have been a realistic solution and the births from these technologies have grown by 5-10% per annum over the last few years (ESHRE, 2014; https://www.ehre.eu/Guidelines-and-Legal/ART-fact-sheet.aspx).

However, only about a third of ART cycles result in live births, increasing the number of cycles that couples required for having a baby and, consequently, the treatments' cost (ESHRE, 2014). One of the most important inconveniences derived from these techniques is to achieve a multiple pregnancy, being associated with risks including premature birth, low birth weight and a dramatic increase in the relative risk of cerebral palsy. To avoid the potential complications regarding multiple pregnancies, the recommendation is to transfer a unique embryo per cycle since it minimizes the risk to have multiple pregnancies after these treatments. Therefore, the selection of the best embryo to transfer is crucial and is becoming one of the most important challenges in the field of ART.

Nowadays, the embryo selection is based on morphokinetic parameters, although most of them do not have a predictive value for ongoing pregnancies. Taking this into an account, complementary procedures based on genetic screening (in situ fluorescence hybridation: FISH) have been developed for the best embryo selection. However, this is an invasive methodology and it is necessary to remove from the embryo one or two blastomeres for diagnostic, compromising the embryo survival. Therefore, it is necessary to develop non-invasive methodologies able to increase the embryo quality for the unique embryo selection with the highest possibilities to achieve a baby at home.

From the point of view of the male, 20%-30% of the men with normal parameters have impaired fertility and inability to achieve pregnancy, suggesting that the predictive value of a semen analysis to identify fertile or infertile males is far from absolute. Semen quality has traditionally been measured under a global viewpoint, following the recommendations established by the World Health Organization (WHO) and based on morphological and physiological parameters. However, semen analysis, performed without adequate quality control is of almost no clinical value. Male infertility, therefore, could be caused by different deficiencies not described previously. Accordingly, improvements may be achieved once robust sperm quality indicators have been established. In this regard, it has been suggested that spermatozoa are more than carriers of genetic material but that some molecular features of the sperm are also involved in fertilization and embryo development. Therefore, implementing new sperm diagnostic and/or selection techniques could significantly improve live birth rates during ART. Today, some studies are focused on developing new diagnostic and/or prognostic sperm biomarkers, but, unfortunately, most of them result in sample destruction, avoiding the possibility to be used during ART. Regarding sperm selection, it has been proposed the use of sperm DNA integrity biomarkers or biomarkers related to sperm function, such as phosphatidylserine and hyaluronic acid respectively, for sperm selection (Nasr-Esfahani et al., J Assist. Reprod. Genet. 2008, 25:197-203; Huszar et al., Reprod. Biomed. Online 2007, 14:650-63; Said and Lan. Hum. Reprod. Update. 2011, 17:719-33; Gil et al, 2013 J. Assist Reprod Genet. 2013, 30:479-85; Nadalini et al., J. Assist. Reprod. Genet. 2014, 31:1045-51; Romany et al, Fertil. Steril., 2014, 102:1567-75, McDowell et al., Cochrane Database Syst. Rev. 2014, 28;10). Although these procedures do not result in sample destruction, their relevance regarding the improvement on ART outcome is still controversial.

Therefore, there is still a need in the art to provide alternative and effective methods for selecting a spermatozoid cell population suitable for ART that ameliorates pregnancy rates.

Gianzo, 2016 discloses the expression of angiotensin II type 2 receptor (AT2R) in human sperm cells and involvement in sperm motility. Human spermatozoa express AT2R mainly at the equatorial segment of the sperm head. The percent of AT2R positive cells correlates negatively with and significantly with sperm concentration and progressive motility, both in fresh semen samples, in prepared sperm cells and in sperm pathologies. Its expression level is associated with sperm motility parameters what makes AT2R a helpful biomarker for selection of the most motile spermatozoa for IVF (Gianzo et al., Fertil Steril. 2016, 105(3):608-616).

Subiran, 2010 reports the implication of neutral endopeptidase (NEP/CD10) and aminopeptidase N (APN/CD13) in motility of human capacitated spermatozoa. These two enzymes are known to be expressed in human sperm cells. Human sperm cells isolated by discontinuous Percoll gradient followed up by swim -up techniques exhibited higher motility upon inhibition with leuhistin, a CD13 specific inhibitor, and thiorphan, a CD10 specific inhibitor. The authors conclude that both inhibitors are useful for sperm activation at different functional stages of spermatozoa (Subiran et al., Asian J Androl. 2010, 12(6):899-902).

Wang, 2016 is a review on in vitro fertilization (IVF) as performed until 2006 (Wang et al., Ther Clin Risk Manag. 2006, 2(4):355-364).

### Summary of the Invention

The authors of the present invention have surprisingly found that the plasma membrane CD10 protein is a suitable biomarker for obtaining a spermatozoid cell population with improved fitness, which is adequate for fertilization.

The scope of the invention is determined solely by the appended claims; any content in the specification not explicitly claimed is intended for illustrative purposes only.

Therefore, in a first aspect the invention relates to a method for obtaining a spermatozoid cell population enriched in CD10⁻ spermatozoids from a starting spermatozoid population comprising:
(i) contacting the starting spermatozoid population with a CD10-binding agent,
(ii) isolating spermatozoids from the starting population which do not bind to the CD10-binding agent.

In another aspect, the invention relates to the use of a CD10-binding agent for the preparation of a spermatozoid cell population enriched in CD10⁻ spermatozoids, for the preparation of a spermatozoid cell population having a high degree of DNA integrity or for the preparation of a spermatozoid cell population having a low level of aneuploidies.

In another aspect, the invention relates to a method for determining the fitness of a human sperm population for fertilization, the DNA integrity of a human sperm population or the number of aneuplodies in a human sperm population which comprises determining the content of CD10⁻ spermatozoids in a said population, wherein increased levels of CD10⁻ spermatozoids in the population with respect to a reference value are indicative that the human sperm is adequate for fertilization, that the sperm population shows a high degree of DNA integrity or that the sperm population shows a low level of aneuploidies.

### Brief description of the figures

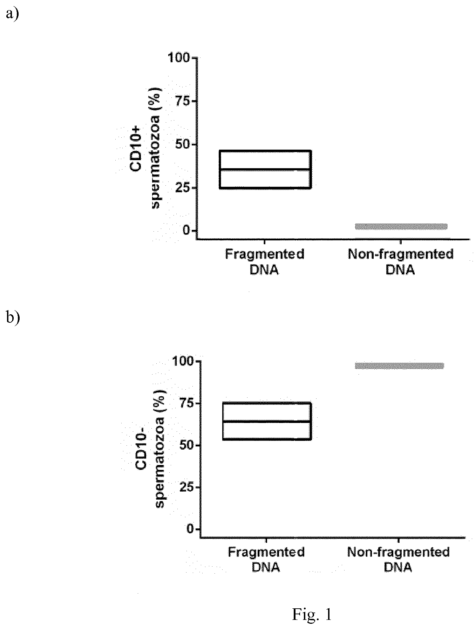

**Figure 1****.** Comparison of sperm subpopulations based on CD10 biomarker in semen samples with fragmented and non-fragmented sperm DNA. The percentage obtained by flow cytometry from a) CD10⁺ and b) CD10⁻ spermatozoa is represented in semen samples with high sperm DNA fragmentation measured with the Halosperm^{®} kit (HALOTECH) to which DGC has been performed to separate fragmented and non-fragmented DNA fractions. The box plot shows the median, the interquartile range and the maximum and minimum values. Mann-Whitney U, p < 0.05.

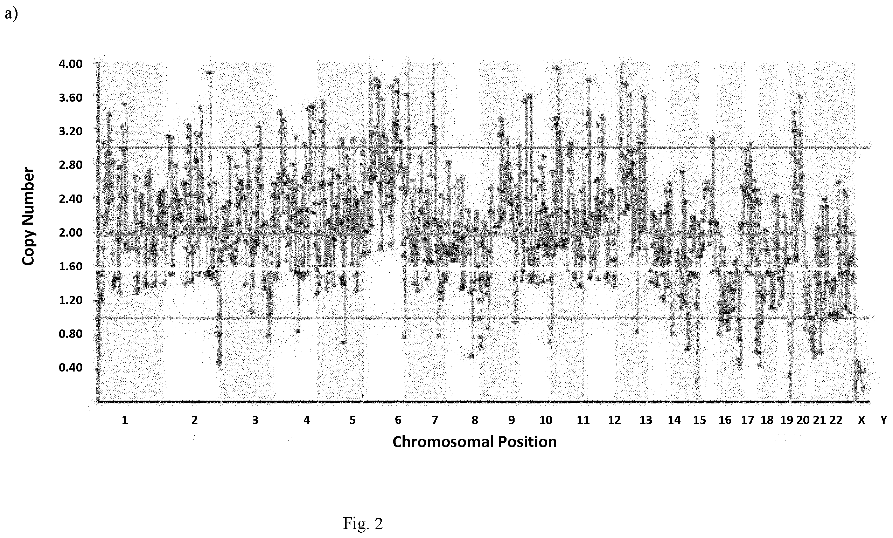

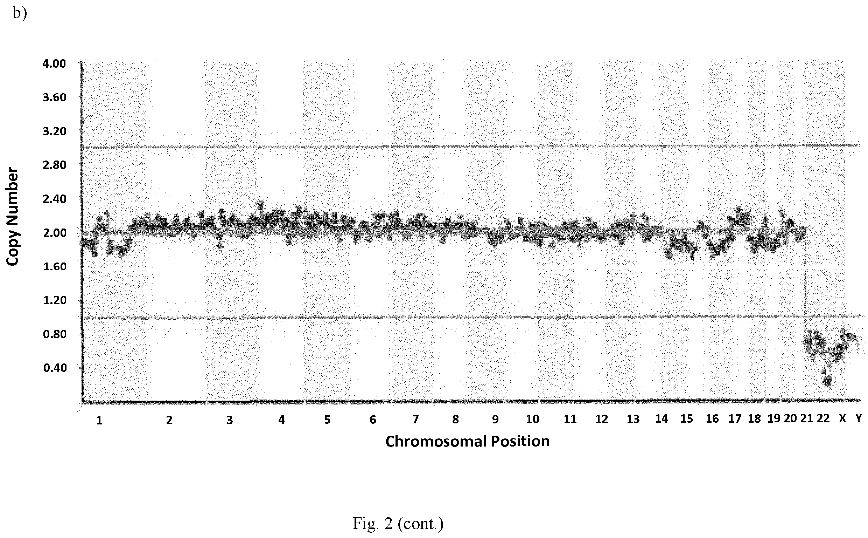

**Figure 2****.** Karyotyping of sperm subpopulations based on CD10 biomarker determined by Next Generation Sequencing (NGS). The karyotype of a) CD10⁺ and b) CD10⁻ spermatozoa is represented.

### Detailed Description of the Invention

### Method for obtaining a spermatozoid cell population enriched in CD10⁻ spermatozoids

The inventors of the present invention have found that a spermatozoid population selected based on the lack of expression of the CD10 surface marker on the spermatozoids are useful in ART methods. Accordingly, in one aspect, the invention relates to a method for obtaining a spermatozoid cell population enriched in CD10⁻ spermatozoids from a starting spermatozoid population comprising:
(i) contacting the starting spermatozoid population with a CD10-binding agent,
(ii) isolating spermatozoids from the starting population which do not bind to the CD10-binding agent.

As used herein, the terms "spermatozoids", "spermatozoa" or "sperm" refer to male gametes.

As used herein, the term "spermatozoid cell population" refers to a spermatozoid population obtained from a semen sample isolated from a subject.

"CD10" or "CD10 antigen" as used herein is referred to the protein Neprylisin, also known as membrane metallo-endopeptidase (MME), neutral endopeptidase (NEP), cluster of differentiation 10 (CD10), and common acute lymphoblastic leukemia antigen (CALLA). As disclosed herein, the presence or absence of CD10 is detected in a spermatozoid cell population. In a preferred embodiment, the spermatozoid cell population is obtained from a mammal. In a preferred embodiment the mammal is human, porcine, bovine, equine, canine, murine or feline. In a more preferred embodiment, the mammal is a human, wherein CD10 is the protein with the sequence shown in the database Uniprot with the accession number: P08473 (date 22 November 2017). In a more preferred embodiment, CD10 is used to obtain an isolated spermatozoid population enriched in CD10⁻ spermatozoids in relation to a starting spermatozoid population.

As used herein, the term "CD10⁻ spermatozoid" refers to spermatozoids which do not express CD10 or which, if they express CD10, it is found at a level which is below the detection level of the techniques and reagents used in the art to detect CD10.

"Enriched" as used in the present invention relates to a spermatozoid cell population obtained after selection which has an increased percentage of CD10⁻ spermatozoids compared to the percentage of CD10⁻ spermatozoids in the starting spermatozoid cell population. In a preferred embodiment, the CD10⁻ spermatozoid cell population is increased by at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, at least 150%, at least 160%, at least 170%, at least 180% or more in CD10⁻ spermatozoids in comparison with the starting spermatozoid cell population. In a more preferred embodiment, the spermatozoid cell population is increased in CD10⁻ spermatozoids by 110%.

In one embodiment, the spermatozoid cell population that is to be enriched in CD10⁻ spermatozoids is a semen sample. As used herein, the term "semen" is used to define a male reproductive fluid comprising spermatozoa and fluid from the seminal vesicles and fluid from the prostate gland and other reproductive glands.

In an embodiment, the spermatozoid cell population that is to be enriched in CD10⁻ spermatozoids is obtained after a primary selection based on morphofunctional analysis based on counting in the starting population the number of sperm cells having normal motility (movement) and morphology (shape) and evaluation of the chemical and biochemical characteristics (e.g., pH, color, turbidity, liquefaction and viscosity) of the population.

As disclosed herein the term "binding agent" refers to a molecule that can specifically and selectively bind to a second (i.e., different) molecule of interest. The interaction may be non-covalent, for example, as a result of hydrogen-bonding, van der Waals interactions, or electrostatic or hydrophobic interactions, or it may be covalent. A binding agent may be soluble, meaning that it refers to a binding agent that is not associated (i.e., covalently or non-covalently bound) to a solid support or associated to a solid support. Non-limiting examples of binding agents include: bioactive peptides, inhibitors, natural ligands and other surface proteins. In a preferred embodiment the CD10-binding agent is an antibody. As used herein, the term "antibody" refers to a protein including at least one immunoglobulin variable region, for example, an amino acid sequence providing an immunoglobulin variable domain or a sequence of the immunoglobulin variable domain. An antibody can include, for example, a variable heavy chain (H) region (herein abbreviated as VH) and a variable light chain (L) region (herein abbreviated as VL). Typically, an antibody includes two variable heavy chain regions and two variable light chain regions. The term "antibody" encompasses antigen-binding antibody fragments (for example, single-chain antibodies, Fab fragments, F(ab')2 fragments, Fd fragments, Fv fragments and dAb fragments) as well as whole antibodies, for example, intact and/or full length immunoglobulins of the IgA, IgG types (for example, IgG1, IgG2, IgG3, IgG4), IgE, IgD, IgM (as well as subtypes thereof). In an embodiment the CD10 antibody can be recognized by a secondary antibody. In a preferred embodiment, the secondary antibody is bound to a fluorophore. As used herein, the term "fluorophore" means a compound or group that fluoresces when exposed to and excited by a light source, such as visible or infrared light. In another preferred embodiment the anti-CD10 antibody is bound to beads. In a more preferred embodiment the anti-CD10 antibody is bound to magnetic beads. As used herein, the term "magnetic beads" means that the beads are susceptible to movement by a magnetic field or create a magnetic field themselves.

Methods to isolate CD10⁻ spermatozoids from a starting population using CD10-binding agents include any technique common in the art used for cell sorting. It will be understood that any of the techniques known in the art can be used both for the isolation of a cell population enriched in cells expressing a marker of interest by selecting those cells which bind to the binding agent of interest but also for the isolation of cell populations enriched in cells which do not express the marker of interest. In this case, the cell population is obtained by selecting those cells which do not bind to the binding agent of interest.

Such techniques include, without being limited to, any cell separation technique based on antigen-antibody interaction. Non-liming examples include: flow cytometry, fluorescence activated cell sorting, magnetic-activated cell sorting (MACS), buoyancy activated cell sorting, immunomagnetic sorting. In a preferred embodiment, spermatozoids are sorted by magnetic-activated cell sorting (MACS). As known in the art, MACS is a method for separation of various cell populations depending on their surface antigens which uses superparamagnetic nanoparticles and columns. The superparamagnetic nanoparticles are used to tag the targeted cells in order to capture them inside the column. The column is placed between permanent magnets so that when the magnetic particle-cell complex passes through it, the tagged cells can be captured. The column consists of steel wool which increases the magnetic field gradient to maximize separation efficiency when the column is placed between the permanent magnets. In one embodiment, nanoparticles are conjugated to a binding agent which can be used to target specific cells. In a preferred embodiment, the binding agent is CD10-binding agent. In a more preferred embodiment, the CD10-binding agent is an antibody. In a preferred embodiment, MACS is used to isolate a spermatozoid cell population enriched in CD10⁻ spermatozoids for not being able to bind to the binding agent.

The term "subject" or "patient", as used herein, refers to all animals classified as mammals and includes, but is not restricted to the Order Rodentia, such as mice; Order Logomorpha, such as rabbits; more particularly the Order Carnivora, including Felines (cats) and Canines (dogs); even more particularly the Order Artiodactyla, Bovines (cows) and Suines (pigs); and the Order Perissodactyla, including Equines (horses); and most particularly the Order Primates, Ceboids and Simoids (monkeys) and Anthropoids (humans and apes). The mammals of preferred embodiments are human, porcine, bovine, equine, canine, murine or feline, preferably humans, either male or female. Preferably, the subject is infertile. In a more preferred embodiment, the subject is infertile due to idiopathic infertility.

In a preferred embodiment, the starting spermatozoid population is isolated from a subject which is infertile. For example, in men, reasons for sub-fertility or infertility include, but are not limited to, infections, hormonal problems in the pituitary gland or the testicles, testicular injury or failure, hematospermia, cancer treatments (e.g., chemotherapy, radiation, etc.), antibodies against sperm (e.g., following vasectomy or injury) or drug use (e.g., tobacco, marijuana, etc.)

In additional embodiments, the starting spermatozoid population is isolated from a subject which is normozoospermic, asthenozoospermic, teratozoospermic, oligozoospermic or any combination thereof.

The term "normozoospermia" refers to a subject afflicted by male infertility associated with apparently normal sperm parameters in seminogram and in sperm morphology characterization (spermocytogram).

The term "asthenozoospermia" refers to spermatozoa of very weak mobility or none at all.

The term "teratozoospermia" refers to a spermocytogram with less than 40% morphologically normal sperm. A sperm concentration of less than 15 million/ml with adequate motility and morphology is considered to be a low sperm count; males exhibiting such characteristics are considered to be oligozoospermic, and the term referred to this condition is "oligozoospermia". The "oligozoospermia" is considered to be severe when sperm count is lower than 5 million/ml. The term "azoospermia" refers to the medical condition of males not having any measurable level of sperm in their semen. In a preferred embodiment, the starting spermatozoid cell population is obtained from a mammal. In a more preferred embodiment, the starting spermatozoid cell population is of human, porcine, bovine, equine, canine, murine or feline origin, preferably human. In a more preferred embodiment, the starting spermatozoid cell population is obtained from an infertile subject. In a still more preferred embodiment the subject is normozoospermic.

### A spermatozoid cell population enriched in CD10⁻ spermatozoids (not covered by the claimed invention)

The inventors of the present disclosure have isolated a spermatozoid cell population enriched in CD10⁻ spermatozoids. Accordingly, although not covered by the claimed invention, the disclosure relates to a spermatozoid cell population enriched in CD10⁻ spermatozoids.

The spermatozoid cell population enriched in CD10⁻ spermatozoids is substantially devoid of CD10⁺ spermatozoids. The content of CD10⁺ spermatozoids in the population is of less than 50%, less than 40%, less than 30%, less than 20%, less than 10%, less than 9%, less than 8%, less than 7%, less than 6%, less than 5%, less than 4%, less than 3%, less than 2%, less than 1%, less than 0,9%, less than 0,8%, less than 0,7%, less than 0,6%, less than 0,5%, less than 0,4%, less than 0,3%, less than 0,2%, less than 0,1%, than 0,05%, less than 0,01 or lower. Preferably, the content of CD10⁺ spermatozoids in the population is of less than 1%.

The spermatozoid cell population enriched in CD10⁻ spermatozoids contains almost exclusively CD10⁻ spermatozoids. The content of CD10⁻ spermatozoids in the population is of at least 50%, at least 60%, at least 70%, at least 80%, at least 90%, at least 91%, at least 92%, at least 93%, at least 94%, at least 95%, at least 96%, at least 97%, at least 98%, at least 99%, at least 99,1%, at least 99,2%, at least 99,3%, at least 99,4%, at least 99,5%, at least 99,6%, at least 99,7%, at least 99,8%, at least 99,9%, at least 99,99 or more. Preferably, the content of CD10⁻ spermatozoids in the population is of at least 99%.

The spermatozoid population is enriched in CD10⁻ spermatozoids by at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, at least 150%, at least 160%, at least 170%, at least 180% or more in comparison with the starting spermatozoid cell population. Preferably, the spermatozoid population is enriched in CD10⁻ spermatozoids by 110%.

The spermatozoid population is enriched in CD10⁻ spermatozoids by at least 2-fold, 4-fold, 6-fold, 8-fold, 10-fold, 20-fold. 30-fold, 40-fold, 50-fold, 60-fold, 70-fold, 80-fold, 90-fold, 100-fold, 1000-fold, 10⁴-fold, 10⁵-fold, 10⁶-fold or more.

The CD10⁻ spermatozoid cell population derives from a mammal. The CD10⁻ spermatozoid cell population is of human, porcine, bovine, equine, canine, murine or feline origin, preferably human.

### In vitro method for obtaining an embryo (not covered by the claimed invention)

The authors of the present disclosure have found that the spermatozoid cell population enriched in CD10⁻ spermatozoids obtained as disclosed herein are particularly useful for obtaining embryos using ART methods. Thus, although not covered by the claimed invention, the disclosure relates to an *in vitro* method for obtaining an embryo, said method selected from the group consisting of:
(i) A method comprising fertilizing an ovum using a spermatozoid population enriched in CD10⁻ spermatozoids or
(ii) A method comprising injecting into an ovum a spermatozoid obtained from the spermatozoid cell population enriched in CD10⁻ spermatozoids or
(iii) A method comprising injecting into an ovum a CD10⁻spermatozoid.

The method to obtain an embryo comprises fertilizing an ovum using a spermatozoid population enriched in CD10⁻ spermatozoids. This is known in the art as *in vitro* fertilization or IVF.

The term "IVF" as used herein refers to a process wherein the ovary releases one or more eggs, optionally as a result of a prior hormone stimulation. The released egg or eggs (oocytes) are collected, maintained in culture medium and the most promising (i.e., most competent) are fertilized by contacting them with sperm obtained from a collected sample. The fertilized eggs (zygotes) are then typically cultured for another 2 (cleavage stage) to 5 (blastocyst stage) days at which time the most promising zygotes (or blastocysts) are transferred to the recipient's uterus, where the implant and continue to grow.

As used herein, the terms, ovum, egg, oocyte, and the like, can be used interchangeably to refer to a female reproductive cell that has not yet been fertilized.

The oocytes come from egg donation, meaning the process by which a woman donates eggs to enable another infertile woman to conceive as part of the application of assisted reproduction technology or for biomedical research. For assisted reproduction purposes, egg donation typically involves *in vitro* fertilization technology, with the eggs being fertilized in the laboratory; more rarely, unfertilized eggs may be frozen and stored for later use. Egg donation is a third party reproduction as part of assisted reproductive technology (ART). The oocytes come from eggs of a woman who freezes her own oocytes before suffering a treatment to cure a pathology that may cause sterility, such as ovary cancer, in order to use them for herself in an ulterior ART.

The method for obtaining an embryo comprising injecting into an ovum a spermatozoid obtained from the spermatozoid cell population enriched in CD10⁻ spermatozoids. This method is known in the art as intracytoplasmic sperm injection (ICSI). The term "intracytoplasmic sperm injection (ICSI)" refers to an *in vitro* fertilization procedure in which a single spermatozoid is injected directly into an oocyte. This procedure is most commonly used to overcome male infertility factors, although it may also be used to overcome female infertility where oocytes cannot easily be penetrated by sperm, and occasionally as a method of *in vitro* fertilization.

The method for obtaining an embryo comprises injecting into an ovum a single spermatozoid cell obtained from the spermatozoid cell population enriched in CD10⁻ spermatozoids. Preferably, the single spermatozoid cell is CD10⁻.

The presence or absence of CD10 in the spermatozoid can be determined by any technique known in the art.

Reference herein to "embryo" is intended to include a blastula, blastocyst, fertilized ovum or an organism in its early stages of development, especially before it has reached a distinctively recognizable form that is to be implanted into a female recipient. The terms are used interchangeably. Preferably, the embryo is a blastocyst. As used herein, the term "blastocyst" refers to the structure formed in the early embryogenesis of mammals, after the formation of the morula. It possesses an inner cell mass (ICM), or embryoblast, which subsequently forms the embryo, and an outer layer of cells, or trophoblast, which later forms the placenta. The trophoblast surrounds the inner cell mass and a fluid-filled blastocyst cavity known as the blastocoele. Preferably, the blastocyst belongs to a mammal. More preferably, the blastocyst origin is human, porcine, bovine, equine, canine, murine or feline, preferably human.

Further, although not covered by the claimed invention, the disclosure relates to an *in vitro* method for obtaining an embryo comprising:
(i) contacting a starting spermatozoid population with a CD10⁻binding agent,
(ii) isolating spermatozoids from the starting population which do not bind to the CD10-binding agent thereby obtaining a spermatozoid cell population enriched in CD10⁻ spermatozoids and
(iii) fertilizing an ovum using a CD10⁻ enriched spermatozoid cell population.

Additionally, although not covered by the claimed invention, the disclosure relates to an *in vitro* method for obtaining an embryo comprising:
(i) contacting a starting spermatozoid population with a CD10-binding agent,
(ii) isolating spermatozoids from the starting population which do not bind to the CD10-binding agent thereby obtaining a spermatozoid cell population enriched in CD10⁻ spermatozoids and
(iii) injecting into an ovum a spermatozoid from the CD10⁻ enriched spermatozoid cell population obtained in step (ii).

The terms "spermatozoids", "spermatozoa", "sperm", "spermatozoid cell population", CD10, CD10⁻ spermatozoid, enriched, "binding agent" and "subject" have been defined in detail in the context of the method for obtaining a spermatozoid cell population enriched in CD10⁻ spermatozoids. Preferably, the spermatozoid from the CD10⁻ enriched spermatozoid cell population used in step (iii) is a CD10⁻ spermatozoid.

The CD10-binding agent is an anti-CD10 antibody. The isolation of CD10⁻ spermatozoid is carried out by magnetic-activated cell sorting. The starting spermatozoid population used for the isolation has been obtained from a subject which is infertile, normozoospermic, asthenozoospermic, teratozoospermic, oligozoospermic or any combination thereof. The starting spermatozoid cell population is of a mammal. Preferably, the mammal is a human, porcine, bovine, equine, canine, murine or feline.

### Insemination method (not covered by the claimed invention)

The authors of the present disclosure have found that the spermatozoid population enriched in CD10⁻ spermatozoids are particularly useful in intrauterine insemination as they lead to embryos showing an improved pregnancy rate. Thus, although not covered by the claimed invention, the disclosure relates to a method comprising the administration into the reproductive tract of a female subject a spermatozoid cell population as described herein. This method is known in the art as "Intrauterine Insemination" or "IUI".

Further, the disclosure relates to a method not covered by the claimed invention comprising:
(i) contacting a starting spermatozoid population with a CD10-binding agent,
(ii) isolating spermatozoids from the starting population which do not bind to the CD10-binding agent thereby obtaining a spermatozoid cell population enriched in CD10⁻ spermatozoids and
(iii) administering into the reproductive tract of a female subject the spermatozoid cell population obtained in step (ii).

This method is also known in the art as "Intrauterine Insemination" or "IUI".

The terms "spermatozoids", "spermatozoa", "sperm", "spermatozoid cell population", CD10, CD10⁻ spermatozoid, enriched, "binding agent" and "subject" have been defined in detail in the context of the method for obtaining a spermatozoid cell population enriched in CD10⁻ spermatozoids.

The CD10-binding agent is an anti-CD10 antibody. The isolation of CD10⁻ spermatozoid is carried out by magnetic-activated cell sorting. The starting spermatozoid population used for the isolation has been obtained from a subject which is infertile, normozoospermic, asthenozoospermic, teratozoospermic, oligozoospermic or any combination thereof. The starting spermatozoid cell population is of a mammal. Preferably, the mammal is a human, porcine, bovine, equine, canine, murine or feline.

The female subject is infertile. Non-limiting causes of female infertility are polycystic ovary syndrome (PCOS), endometriosis, poor egg quality, Poor Responders to Ovarian Stimulation or low ovarian reserve.

"Polycystic ovary syndrome" (PCOS) as used herein relates to a common health problem caused by an imbalance of reproductive hormones. Signs and symptoms of PCOS include irregular or no menstrual periods, heavy periods, excess body and facial hair, acne, pelvic pain, difficulty getting pregnant, and patches of thick, darker, velvety skin. Associated conditions include type 2 diabetes, obesity, obstructive sleep apnea, heart disease, mood disorders, and endometrial cancer.

"Endometriosis" as used herein is a condition in which the layer of tissue that normally covers the inside of the uterus grows outside of it. Most often this is on the ovaries, fallopian tubes, and tissue around the uterus and ovaries; however, in rare cases it may also occur in other parts of the body. The main symptoms are pelvic pain and infertility.

"Poor egg quality" as used herein means an egg is not viable enough to conceive a healthy baby. Non-limiting causes of poor egg quality include: maternal age, certain medications or medical treatments and smoking.

The term "poor responder" as used herein is referred to someone whose ovaries and body does not respond to fertility medications. Usually they will require much higher doses of stimulation medications to produce 4 or less than optimal number of eggs needed to proceed with an in vitro fertilization treatment.

"Low ovarian reserve" as used herein means a condition where there is a physiological decrease in the number of eggs, resulting in an insufficient number to ensure a reasonable chance of pregnancy. Generally, it is caused by aging ovaries.

Preferably, the female subject suffers of polycystic ovary syndrome (PCOS), endometriosis, poor egg quality, Poor Responder to Ovarian Stimulation or low ovarian reserve.

The CD10⁻ spermatozoid population is administered in the uterus of the female subject. The female subject is preferably a mammal, more preferably, of human, porcine, bovine, equine, canine, murine or feline origin, preferably human.

Infertility is due to male infertility, preferably, idiopathic infertility. The starting spermatozoid population is normozoospermic, asthenozoospermic, teratozoospermic, oligozoospermic or any combination thereof. Preferably, the starting spermatozoid population is normozoospermic.

The terms "ovum" or "oocyte" are used interchangeably herein and refer to the gamete from the follicle of a female animal. Preferably, the animal is a mammal. More preferably, the mammal is human, porcine, bovine, equine, canine, murine or feline, more preferably a human. "Immature" oocytes refer to oocytes that are viable but incapable of fertilization without additional growth or maturation. Oocytes recovered from "unstimulated" follicles or ovaries are natural oocytes obtained from follicles or ovaries that were not treated with any gonadotropins or other hormones or agents to stimulate maturation of the oocytes. Oocytes recovered from "stimulated" ovaries may be either mature or immature. Subjective criteria to estimate the viability and maturity of the ovum that can be done microscopically after removal of the ovum from the follicle include assessing the number and density of surrounding granulosa cells, the presence or absence of the germinal vesicle, and the presence or absence of the first polar body. The oocytes belong to the same cohort, preferably wherein all of them come from the same patient. The female subject is in a reproductive age, between menarche and menopause. Preferably, the female subject is treated for ovarian stimulation and oocyte production.

Both the methods for obtaining an embryo as disclosed herein and the method for insemination as disclosed herein can be used in order to achieve pregnancy in infertile couples or individuals.

### Embryo obtained by in vitro fertilization (not covered by the claimed invention)

The authors of the present disclosure have found that embryos obtained by *in vitro* fertilization using a spermatozoid cell population enriched in CD10⁻ spermatozoids show improved implantation rates. Thus, although not covered by the claimed invention, the disclosure relates to an embryo obtained by *in vitro* fertilization of an ovum with a spermatozoid cell population enriched in CD10⁻ spermatozoids. Preferably, the fertilization is achieved by ICSI (Intracytoplasmic Sperm Injection). The disclosure relates to an embryo obtained by a method according to any of the methods for obtaining an embryo as defined previously.

Preferably, the embryo is non-human. More preferably, the embryo is in the blastocyst stage. The embryo is non-human and is found in the blastocyst stage. The embryo is porcine, bovine, equine, canine, murine or feline.

The term "blastocyst", as used herein, refers to the structure formed in the early development of embryo characterized in that it possesses an inner cell mass (ICM) which subsequently forms the embryo, an outer layer of cells collectively called the trophoblast which surrounds the inner cell mass and a fluid-filled cavity known as the blastocoele.

Preferably, the embryo has an increased blastocyst rate.

The term "blastocyst rate", as used herein, defines the ratio of embryos which reach the blastocyst stage. "Increased blastocyst rate", relates to higher percentage of embryos which successfully reach the blastocyst stage when obtained by fertilizing of an ovum using a CD10⁻ enriched spermatozoid population when compared to the percentage of embryos which successfully reach blastocyst stage when using a spermatozoid population which have not been enriched for the absence of CD10 expression. The embryos obtained using the method disclosed herein have a blastocyst rate which is increased by at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, at least 150%, at least 160%, at least 170%, at least 180% or more with respect to the blastocyst rate obtained using a non-enriched spermatozoid population. Preferably, the blastocyst rate is increased 10% with respect to the blastocyst rate obtained using a non-enriched spermatozoid population. Blastocys rate can be measured by any method known in the art such as the method disclosed by Mizobe Y et al., (Reprod Med Biol. 2017; 17:64-70).

### Method for obtaining a blastocyst population with increased implantation rate (not covered by the claimed invention)

The authors of the present disclosure have found that blastocysts obtained by ART methods using a CD10⁻ enriched spermatozoid population as described herein show an unexpected high implantation rate. Accordingly, although not covered by the claimed invention, the disclosure relates to a method for obtaining a blastocyst population with an increased implantation rate wherein the method comprises obtaining an embryo using a method as described herein and allowing the embryo to develop to the stage of blastocyst.

Thus, although not covered by the claimed invention, the disclosure relates to an *in vitro* method for obtaining a blastocyst population with increased implantation rate comprising:
(i) contacting a starting spermatozoid population with a CD10-binding agent,
(ii) isolating spermatozoids from the starting population which do not bind to the CD10-binding agent thereby obtaining a spermatozoid cell population enriched in CD10⁻ spermatozoids ,
(iii) fertilizing an ovum using a CD10⁻ enriched spermatozoid cell population thereby obtaining an embryo and
(iv) allowing the embryo to develop to the stage of blastocyst.

Further, although not covered by the claimed invention, the disclosure relates to an *in vitro* method for obtaining an embryo comprising:
(i) contacting a starting spermatozoid population with a CD10-binding agent,
(ii) isolating spermatozoids from the starting population which do not bind to the CD10-binding agent thereby obtaining a spermatozoid cell population enriched in CD10⁻ spermatozoids,
(iii) injecting into an ovum a spermatozoid from the CD10⁻ enriched spermatozoid cell population obtained in step (ii) thereby obtaining an embryo and
(iv) allowing the embryo to develop to the stage of blastocyst.

The terms "spermatozoids", "spermatozoa", "sperm", "spermatozoid cell population", CD10, CD10⁻ spermatozoid, enriched, "binding agent" and "subject" have been defined in detail in the context of the method for obtaining a spermatozoid cell population enriched in CD10⁻ spermatozoids.

The CD10-binding agent is an anti-CD10 antibody. The isolation of CD10⁻ spermatozoid is carried out by magnetic-activated cell sorting. The starting spermatozoid population used for the isolation has been obtained from a subject which is infertile, normozoospermic, asthenozoospermic, teratozoospermic, oligozoospermic or any combination thereof. The starting spermatozoid cell population is of a mammal. Preferably, the mammal is a human, porcine, bovine, equine, canine, murine or feline.

Implantation rate can be measured using any method known in the art such as the method disclosed in Mersereau J et al., (Fertil Steril. 2017; 108:750-756).

The term "implantation" is used to describe a process of attachment and invasion of the uterus endometrium by the blastocyst (conceptus) in placental animals. "Implantation rate" is the percentage of embryos which successfully undergo implantation compared to the number of embryos transferred in a given period. "Increased implantation rate", as used herein relates to higher percentage of embryos which successfully undergo implantation when isolating spermatozoids according to the method described herein compared to the percentage of embryos which successfully undergo implantation when using unselected spermatozoids. The implantation rate is increased by at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, at least 150%, at least 160%, at least 170%, at least 180% or more. In practice, implantation is generally calculated as the number of intrauterine gestational sacs observed by transvaginal ultrasonography divided by the number of transferred embryos. The implantation as disclosed herein may be a natural implantation or due to an assisted reproductive technology such as artificial insemination, *in vitro* fertilization (IVF), Intracytoplasmic sperm injection (ICSI), or following transplantation of a fresh or frozen or otherwise preserved embryo(s) and gametes, gamete intrafallopian transfer (GIFT) or zygote intrafallopian transfer (ZIPT). Preferably, pregnancy is the consequence of assisted reproductive technology. More preferably, the implantation is preceded by *in vitro* fertilization (IVF), ICSI or IUI using any of the methods explained above.

The method for obtaining a blastocyst with an increased implantation rate comprises obtaining an embryo as described herein and allowing the embryo to develop to the stage of blastocyst.

The method comprises the culture of embryos until they reach stage 1, 2, 3, 4 or 6 according to the blastocyst grading system developed by Gardner and Schoolcraft, (Gardner DK, Schoolcraft WB., In vitro culture of human blastocysts. In: Jansen R, Mortimer D (eds). Toward Reproductive Certainty: Fertility and Genetics Beyond 1999, UK: Parthenon Publishing London, 1999, 378-388.), which is based on the grading of the inner cell mass (A, B and C) as well as for trophoectoderm (grades 1 to 3), in addition to the degree of blastocyst expansion (score 1-6).

Culture of the embryos until they reach the blastocyst stage can be carried out using any method known in the art, including any if the culture conditions and including using any of the culture conditions mentioned by Gardmer (Reproductive BioMedicine Online, 2003, 6:470-481).

### Method for achieving pregnancy (not covered by the claimed invention)

The inventors of the present disclosure have found a method for achieving pregnancy. Thus, although not covered by the claimed invention, the disclosure relates to a method for achieving pregnancy in a female subject comprising transferring into said female subject an embryo obtained after fertilizing an ovum with a CD10⁻ spermatozoid enriched population using any of the methods described herein. In addition, the disclosure also relates to a method for achieving pregnancy in a female subject comprising transferring into said female subject an embryo obtained after fertilizing an ovum with a CD10⁻ spermatozoid.

In addition, the disclosure also relates to a method for achieving pregnancy in a female subject comprising:
(i) contacting a starting spermatozoid population with a CD10-binding agent,
(ii) isolating spermatozoids from the starting population which do not bind to the CD10-binding agent thereby obtaining a spermatozoid cell population enriched in CD10⁻ spermatozoids ,
(iii) fertilizing an ovum using a CD10⁻ enriched spermatozoid cell population thereby obtaining an embryo and
(iv) transferring into said female subject the embryo obtained in step (iii).

"Pregnancy", also known as "gestation", is the time during which one or more offspring develop inside a female subject. Pregnancy is achieved after transferring into said female subject an embryo obtained after fertilizing an ovum with a CD10⁻ spermatozoid enriched population. Preferably, the embryo that is to be transferred into the female mammal is an embryo on day 3 or a blastocyst.

The method for achieving pregnancy is advantageous over other methods known in the art as it results in an improved pregnancy rate.

"Improved pregnancy rate" is intended to include a higher pregnancy outcome or improved perinatal survival or general viability following the transfer of an embryo obtained according to the method disclosed herein in relation to the pregnancy rate obtained in the absence of any embryo selection. The pregnancy rate may be defined in various ways, in an illustrative non limitative example may be based on fetal heart motion observed in ultrasound examination. The pregnancy rate is increased by at least 5%, at least 10%, at least 20%, at least 30%, at least 40%, at least 50%, at least 60%, at least 70%, at least 80%, at least 90%, at least 100%. Preferably, the pregnancy rate is increased between 5-20%. More preferably, the pregnancy rate is increased 5% when embryos are obtained after fertilization of oocytes coming from egg donation. More preferably, the pregnancy rate is increased 20% when embryos are obtained after fertilization of oocytes with ICSI.

Preferably, the female subject is infertile.

"Infertility" as used herein relates to the inability to reproduce by natural means. Particularly, is a disease of the reproductive system defined by the failure to achieve a clinical pregnancy after 12 months or more of regular unprotected sexual intercourse (and there is no other reason, such as breastfeeding or postpartum amenorrhoea). Infertility may be due to male or female infertility, or both. Infertility is idiopathic infertility. As disclosed herein, idiopathic infertility or unexplained infertility means infertility which cause remains unknown even after an infertility study, usually including semen analysis in the male and assessment of ovulation and fallopian tubes in the female. Preferably, female infertility is due to a polycystic ovary syndrome (PCOS), endometriosis, poor egg quality, Poor Responders to Ovarian Stimulation or low ovarian reserve. Infertility is due to male infertility. Preferably, infertility is due to normozoospermia, asthenozoospermia, teratozoospermia, oligozoospermia or any combination thereof. More preferably, the patient is noormozoospermic.

Additionally, infertility can be due to sperm or eggs of poor quality. However, other factors may also affect fertility. For example, in men, reasons for sub-fertility or infertility include, but are not limited to, infections, hormonal problems in the pituitary gland or the testicles, testicular injury or failure, hematospermia, cancer treatments (e.g., chemotherapy, radiation, etc.), antibodies against sperm (e.g., following vasectomy or injury) or drug use (e.g., tobacco, marijuana, etc.). In women, reasons for sub-fertility or infertility include, but are not limited to, infections, scarring of uterus or fallopian tubes, endometriosis, cysts, fibroids, damage to the cervix or uterus (for example, following surgery such as D and C), hormonal problems, abnormalities in cervical mucus, antibodies to sperm, cancer treatment, thyroid problems, stress or drug use (e.g., tobacco, marijuana, etc.).

Preferably, the blastocyst is of non-human origin.

The terms "spermatozoids", "spermatozoa", "sperm", "spermatozoid cell population", CD10, CD10⁻ spermatozoid, enriched, "binding agent" and "subject" have been defined in detail in the context of the method for obtaining a spermatozoid cell population enriched in CD10⁻ spermatozoids.

The CD10-binding agent is an anti-CD10 antibody. The isolation of CD10⁻ spermatozoid is carried out by magnetic-activated cell sorting. The starting spermatozoid population used for the isolation has been obtained from a subject which is infertile, normozoospermic, asthenozoospermic, teratozoospermic, oligozoospermic or any combination thereof. The starting spermatozoid cell population is of a mammal. Preferably, the mammal is a human, porcine, bovine, equine, canine, murine or feline.

### Use of a CD10-binding agent

The inventors of the present invention have found a use for a CD10-binding agent. Thus in another aspect the invention relates to the use of a CD10-binding agent for the preparation of a spermatozoid cell population enriched in CD10⁻ spermatozoids and/or the use of CD10-binding agent for the selection of a CD10⁻ spermatozoid. In further aspects, the invention relates to the use of a CD 10-binding agent for the preparation of a spermatozoid cell population having a high degree of DNA integrity. In another embodiment, the invention relates to the use of a CD10-binding agent for the preparation of a spermatozoid cell population having a low level of aneuploidies.

All the terms and embodiments previously described are equally applicable to this aspect of the invention.

The term "DNA integrity", as used herein, refers to the measure of the content of DNA in a sample of nucleic acid which has been subjected to a fragmentation process such that a statistically significant greater number of single-stranded breaks (SSBs) or double-stranded breaks (DSBs) exist in the sample as compared to prior to the fragmentation process.

The term "aneuploidy" herein refers to an imbalance of genetic material caused by a loss or gain of a whole chromosome, or portion of a chromosome.

In a preferred embodiment the CD10-binding agent is an anti-CD10 antibody.

### Method for determining the fitness of a human sperm population for fertilization, for determining the DNA integrity of a human sperm population or for determining the level of aneuplodies in a human sperm population

The inventors of the present invention have found that the content of CD10⁻ spermatozoids in a spermatozoid cell population is related to the fitness of a sperm population for fertilization, to the degree of DNA integrity and to the level of aneuplodies in the DNA of the cell population. Thus, in further aspects, the invention relates to:
- A method for determining the fitness of a human sperm population for fertilization which comprises determining the content of CD10⁻ spermatozoids in a said population, wherein increased levels of CD10⁻ spermatozoids in the population with respect to a reference value are indicative that the human sperm is adequate for fertilization.
- A method for determining the DNA integrity of a human sperm population which comprises determining the content of CD10⁻ spermatozoids in a said population, wherein increased levels of CD10⁻ spermatozoids in the population with respect to a reference value are indicative that the sperm population shows a high degree of DNA integrity or
- A method for determining the level of aneuplodies in a human sperm population for fertilization which comprises determining the content of CD10⁻ spermatozoids in a said population, wherein increased levels of CD10⁻ spermatozoids in the population with respect to a reference value are indicative that the human sperm population shows low level of aneuploidies.

The term "fitness" or "sperm quality", as used herein, refers to the ability of the spermatozoid population to perform successful fertilization leading to embryo development that is able to give rise to pregnancy.

In a first step, the methods for determining the fitness of a human sperm population for fertilization, for determining the DNA integrity of a human sperm population or for determining the level of aneuplodies in a human sperm population comprise determining the content of CD10⁻ spermatozoids in a said population. The method is carried out essentially as described in the method for obtaining a spermatozoid cell population enriched in CD10⁻ spermatozoids with the difference that the number of CD10⁻ spermatozoids is determined after the negative isolation step. The number of CD10⁻ spermatozoids can be determined using any method known in the art.

The cell population is typically found in a semen sample.

In a preferred embodiment the semen sample is to be understood as a sample comprising semen and/or components derived from semen.

In a preferred embodiment the semen sample is a sample comprising spermatozoa. Rarely in the clinic subjects are seen with semen samples comprising very few or even no spermatozoa in their semen, samples from such subjects is still of interest and thus included by the present definition of the sample base, as such samples would indeed be a sample which would be indicative of low fertility potential.

The semen sample may be obtained after ejaculation, aspiration from the testis, epididymis or after testicular biopsy or microdissection of the testis.

In a particular preferred embodiment the semen sample is a sample comprising spermatozoa and/or components derived from an ejaculate.

Procedures for collecting semen samples from human or animals such as farm animals is well described in the literature and well known for a person skilled in the art.

In another embodiment of the present invention, a minimum of handling steps of the sample is necessary before measuring the content of CD10⁻ spermatozoids.

In the present context, the subject "handling steps" relates to any kind of pre-treatment of the semen sample before determining the content in CD10⁻ spermatozoids. Pre-treatment procedures includes washing, lysis, immunocapture, cytospin, fixation, separation, spin down, filtration, dilution, distillation, concentration, inactivation of interfering compounds, centrifugation, heating, fixation, addition of reagents, or chemical treatment.

In a preferred embodiment the semen sample is an ejaculate. In a preferred embodiment the sample may be up-concentrated by centrifugation using a gradient such as Percoll gradient centrifugation. In another preferred embodiment the sample may be up concentrated by centrifugation without a gradient.

In a presently preferred embodiment said pre-treatment procedures comprises mixing the semen sample with a phosphate buffered saline solution and subsequently spinning down the sample.

In a particular preferred embodiment the freshly delivered semen sample is centrifuged and cellular sediments collected before determining the contents of CD10⁻ spermatozoids. Sperm may be "washed" by density gradient centrifugation or by a "direct swim-up" technique that doesn't involve centrifugation.

In another embodiment the sperm are separated from the seminal fluid before determining the content in CD10⁻ spermatozoids.

In a particular preferred embodiment no pre-treatment of the sample is necessary.

In a particular preferred embodiment the sample is a raw unmodified semen sample. The raw unmodified semen sample may be fresh.

In one embodiment, the sample is a semen sample which may have been stored for several days before determination of the content of CD10⁻ spermatozoids. In a preferred embodiment pre-treatment of the samples such as but not limited to cytospin of the sample prolongs the time the sample can be stored. In a preferred embodiment the samples may be stored for at least one day, such as at least 7 days such as at least 30 days. In another preferred embodiment the samples may be stored for several years before detecting the content of CD10⁻ spermatozoids such as at least one year, such as at least two years such as at least five years such as at least 10 years.

In a second step, the method for determining the fitness of a sperm population for fertilization which comprises comparing the level of CD10⁻ spermatozoids with a reference sample wherein increased levels of CD10⁻ spermatozoids in the population with respect to a reference value are indicative that the human sperm is adequate for fertilization.

"Reference value", as used herein relates to a laboratory value used as a reference for the values/data obtained from samples. The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample, such as, for example, a value obtained from a sample of study but obtained at a previous point in time. The reference value can be based on a high number of samples, such as the values obtained in a population of samples or based on a pool of samples including or excluding the sample to be tested.

In an embodiment the reference value is determined by the number of CD10⁻ spermatozoids in a non-selected spermatozoid cell population.

In a preferred embodiment if the content of CD10⁻ spermatozoids in a non-selected spermatozoid cell population is increased with respect to said reference value, the human sperm is adequate for fertilization. If the content of CD10⁻ spermatozoids in a non-selected spermatozoid cell population is below said reference value, the human sperm is not adequate for fertilization. In a preferred embodiment the content of CD10⁻ spermatozoids is increased with respect to the reference value by at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, at least 150%, at least 160%, at least 170%, at least 180% or more. In a more preferred embodiment the content of CD10⁻ spermatozoids is increased by 110%.

In a preferred embodiment the content of CD10⁻ spermatozoids is determined using a CD10-binding agent. In a more preferred embodiment the CD10-binding agent is an antibody. In a more preferred embodiment the antibody is bound to a fluorophore or magnetic beads. In a more preferred embodiment the CD10⁻ spermatozoid content is determined by flow cytometry.

In an embodiment, human sperm is isolated from a subject which is infertile. In a preferred embodiment, the subject is normozoospermic, asthenozoospermic, teratozoospermic, oligozoospermic or any combination thereof.

In addition, the method of the present invention for determining the fitness of a sperm cell population for fertilization can be combined with one or more parameters which are commonly used in the art known to determine sperm fitness such as sperm concentration in the population, the motility and the morphology of spermatozoa. Motility, morphology and sperm count are considered as markers of fertility and used to predict pregnancy success.

In addition, the method for determining the DNA integrity of a human sperm population can be combined with one or more parameters which are commonly used in the art known to determine sperm fitness such as sperm concentration in the population, the motility and the morphology of spermatozoa. Motility, morphology and sperm count are considered as markers of fertility and used to predict pregnancy success.

In addition, the method for determining the level of aneuplodies in a human sperm population can be combined with one or more parameters which are commonly used in the art known to determine sperm fitness such as sperm concentration in the population, the motility and the morphology of spermatozoa. Motility, morphology and sperm count are considered as markers of fertility and used to predict pregnancy success.

As used in the present invention, the term "sperm quality" also refers to infertility index, sperm proteome integrity, or spermatozoa proteome quality index (SPQI).

All the terms and embodiments previously described are equally applicable to this aspect of the invention.

The invention is described below by way of the following examples which are to be construed as merely illustrative and not limitative of the scope of the invention.

### Examples

### Materials and methods

### Study design

The invention is related to the use of the surface membrane protein CD10 as a biomarker for sperm selection. The Magnetic-activated cell sorting Microbeads MACS^{©} form Miltenyi Biotec Company has been used to select CD10 negative human spermatozoa for ART. Specifically, the commercial kit "CD10 MicroBead Kit, human". MACS MicroBeads is based on small superparamagnetic particles (50-nm) conjugated to highly specific antibodies against a particular antigen. Due to the small size, they do not modify the structure, function or cell activity, and they will not saturate cell surface epitopes. MACS MicroBeads are biodegradable and not toxics. Cells in a single-cell suspension are magnetically labelled with MACS MicroBeads. The sample is applied to a MACS Column that contains a matrix composed by ferromagnetic spheres and placed in magnetic separator.

The unlabeled cells pass through the column while the magnetically labelled cells are retained within the column. The flow-through can be collected as the unlabeled cell fraction.

Human Sperm Selection will carry out in two phases:
a).- Binding: Anti-CD10 antibody are first bound to de MACS^{©} microbeads and then incubated with human sperm cells to recognize its corresponding epitope. CD10-positive spermatozoa are labelled with anti-CD10 antibody conjugated with magnetic microbeads.
b).- Separation: Cell suspension pass through MACS Column placed on a magnetic separator. The unlabeled CD10 spermatozoa cells pass through while the magnetically labeled CD10 spermatozoa are retained within the column.

Specifically, isolated spermatozoa obtained after swim-up procedure (10 million of sperm of post-swim-up fraction) was incubated with 10 µL of anti-CD10 conjugated with biotin (10 minutes, room temperature). Next, 20 µL of anti-biotin MicroBeads was added and incubated for 15 minutes at room temperature. The sperm/microbeads suspension was loaded into a separation column coated with a cell-friendly matrix containing iron spheres, which was fixed with a magnet (MiniMACS; MiltenyiBiotec).The fraction of CD10-positive spermatozoa (CD10+) was retained in the separation column, and the CD10-negative sperm fraction (CD10-) was eluted through the column. The negative selected CD10 human sperm fraction will be ready for oocyte fertilization.

A prospective, randomized and controlled cohort study (n=54) to evaluate the use of the surface membrane protein CD10 as a biomarker for human sperm selection during ART has been carried out. The primary endpoint of this study was pregnancy rate after intracytoplasmic sperm injection (ICSI), with and without the use of CD10 for sperm selection in couples undergoing ICSI treatments with their own oocytes without any apparent female pathology (PO) and in couples undergoing ovum donation (OD) to avoid the bias associated with oocyte quality in female infertility. Half oocytes from the same oocyte cohort was fertilized with CD10+ (semen samples prepared by swim-up: Goal standard) and the other half oocyte cohort with CD10- sperm samples (semen samples prepared by swim-up followed by CD10 MACS isolation). Paired ART outcomes per pairs were evaluated.

### Purification of sperm subpopulations based on CD10 biomarker in semen samples subjected to different procedures.

The percentage of CD10⁺ and CD10⁻ was obtained by flow cytometry from spermatozoa in unprocessed semen samples (N = 8), processed by conventional methods of sperm capacitation such as swim-up (SU; N = 14) and density gradient centrifugation (DGC; N = 5) and subjected to magnetic-activated cell sorting by CD10, in the Miltenyi (MACS CD10; N = 9) platform.

### Results

The number of couples included in this study was 54 divided into 28 couples from OD and 26 couples from PO. A total of 756 MII-oocytes (Metaphase II oocytes) were recovered. 369 oocytes were fertilized with CD10+ sperm samples and 387 with CD10- sperm samples. After the fertilization assessment, a total of 216 embryos were obtained. 111 embryos came from oocytes fertilized with CD10+ semen samples, while 105 were obtained from CD10- semen samples.

ART outcomes were summarized in table1:

**Table 1: ART outcomes grouping per pairs obtained from the same oocyte cohort fertilized with CD10⁺ and CD10- sperm samples, taking into an account the total number of couples undergoing intracytoplasmic sperm injection (ICSI) treatments (ALL; n=54), couples undergoing treatment with ovum donation (OD; n=28) and couples undergoing treatment with their own oocytes (PO; n=26).**

| **CD10+ Sperm sample** | **Fertilization rate %** | **Blastocyst rate %** | **Transfer rate %** | **Pregnancy rate%** | **Implantation rate %** |
|---|---|---|---|---|---|
| **ALL** | 82.11 | 54.95 | 42.34 | 23.33 | 14.89 |
| **OD** | 84.90 | 58.57 | 40.00 | 35.29 | 21.43 |
| **PO** | 79.10 | 48.78 | 46.34 | 7.69 | 5.26 |

| **CD10-Sperm sample** | Fertilization rate % | Blastocyst rate % | Transfer rate % | Pregnancy rate% | Implantation rate % |
|---|---|---|---|---|---|
| **ALL** | 83.20 | 64.76 | 42.86 | 32.26 | 22.22 |
| **OD** | 82.80 | 73.47 | 32.65 | 41.67 | 31.25 |
| **PO** | 83.58 | 57.14 | 51.79 | 26.32 | 17.24 |

- Fertilization rates: no difference between groups. The fertilization rates were around 80% in both cases.
- Blastocyst rate: taking into an account that blastocysts have a higher implantation potential than early embryos, the blastocyst rate has been considered as an embryo quality indicator. The negative selection of CD10 human spermatozoa increased 10% the blastocyst rate (64.76%) compared with the embryos obtained from semen samples prepared only by swim-up (54.95%). In addition, we observed an increase of 15% in OD, when the female factor was avoided, and an increase of 9% in PO couples.
- Transfer rate: the selection of the best embryo to transfer was based on morfokinetic parameters at the blastocyst stage taking into an account the inner mass cell and trophoectoderm quality. Although, there are no differences in transfer rates between both groups considering the total number of couples in this study (CD10+: 42,34% and CD10-: 42,86%), there was a decrease of 8% on transfer rates in OD and an increase of 5% in PO.
- Pregnancy rate: although there are no differences in transfer rates, there is an increase of 9% on pregnancy rates using embryos came from CD10- sperm samples. Specifically, an increase of 5% on pregnancy rates using CD10 negative selected spermatozoa, in spite of a decrease on the total embryos transferred in this group. Regarding PO couples, the negative selection of CD10-spermatozoa led to an increase of 20% in pregnancy rates, consistent with the increase on transfer rates observed when embryos were fertilized with CD10 negative spermatozoa.
- Implantation rate. Consistent with pregnancy rates, an increase of 8% on implantation rate was observed using embryos obtained from CD10 negative sperm samples (22.22%) compared to embryos obtained from CD10+ sperm samples (14.89%). A greater increase was observed in PO couples (15%) than in OD couples (10%). In both groups, the use of embryos obtained after the fertilization with CD10 negative led to this improvement in implantation rates.

To sum up, the negative selection of CD10⁻ spermatozoa increases up to 10% the embryo quality and up to 20% the pregnancy rates during ART. One important advantage of this invention is that the marker used for spermatozoid selection (CD10) is found on the surface of the cells. This means that cell separation techniques based on antigen-antibody interaction, such as flow cytometry or immunomagnetic sorting, can be used to select cells not expressing this marker, which will be potentially useful for sperm selection without affecting sperm functionality or cellular viability.

### CD10⁺ sperm population after different sperm selection methodologies

The percentage of CD10⁺ spermatozoa in semen samples varies depending on the processing technique applied. A method based on magnetic-activated cell sorting (MACS CD10) is more efficient in depleting CD10⁺ spermatozoa than conventional methods such as swim-up (SU) and density gradient centrifugation (DGC). The highest percentage of CD10-spermatozoa is obtained by using MACS platform (see table 1).

**Table 1. Flow cytometry analysis of sperm subpopulations based on CD10 biomarker in semen samples subjected to different procedures. The median and interquartile range of the percentages of CD10⁺ and CD10⁻ spermatozoa are represented. SU: swim-up; DGC: density gradient centrifugation; MACS: magnetic-activated cell sorting.**

| | **CD 10⁺ (%)** | **CD10⁻ (%)** |
|---|---|---|
| Fresh semen | 5.79 (2.65 - 18.77) | 94.21 (81.23 - 97.35) |
| DGC | 4.36 (0.24 - 5.35) | 95.64 (94.65 - 99.76( |
| SU | 2.15 (0.66 - 4.78) | 97.85 (95.22 - 99.34) |
| **MACS CD10** | **0.14 (0.13 - 1.01)** | **99.86 (98.99 - 99.87)** |

### CD10⁺ sperm population is related to DNA fragmentation in human spermatozoa

The relationship between the fragmentation of sperm DNA and the distribution of sperm subpopulations based on the CD10 biomarker was studied. Semen samples with high sperm DNA fragmentation measured with the Halosperm^{®} kit (HALOTECH) were analyzed. These samples were subjected to DGC to separate the fragmented from the non-fragmented DNA. Flow cytometry analysis of sperm subpopulations based on CD10 biomarker revealed a higher percentage of CD10⁺ spermatozoa in samples with fragmented DNA [35.68 % (24.9 % - 46.46 %)] compared to those without DNA fragmentation [2.55 % - (1.5 % - 3.6 %); p < 0,05)] (Fig. 1.a), therefore, in samples with fragmented DNA the percentage of CD10⁻ spermatozoa was lower [64.32 % (53.54 % - 75.1 %)] than in samples with non-fragmented DNA [97.45 % (96.4 % - 98.5 %); p < 0,05] (Fig.1.b).

### CD10⁺ sperm population is related to DNA integrity and aneuploidies in human spermatozoa

In the study of the karyotype of CD10⁺ and CD10⁻ spermatozoa by the technique of mass sequencing (Next Generation Sequencing, NGS) it was observed that CD10⁺ spermatozoa presented aneuploidies or chromosomal alterations (Fig. 2.a) while spermatozoa CD10⁻ were euploids (Fig. 2.b).

In conclusion, sperm selection based on the CD10 biomarker provides a population richer in CD10-spermatozoa than conventional sperm capacitation techniques. The population of spermatozoa CD10⁻ obtained presents less DNA fragmentation, greater DNA integrity and absence of aneuploidies, therefore, the CD10 biomarker is a good biomarker for those indicators.

## Claims

1. A method for obtaining a spermatozoid cell population enriched in CD10⁻ spermatozoids from a starting spermatozoid population comprising:
(i) contacting the starting spermatozoid population with a CD10-binding agent and
(ii) isolating spermatozoids from the starting population which do not bind to the CD10-binding agent.

2. The method according to claim 1 wherein the CD10-binding agent is an anti-CD10 antibody.

3. The method according to claim 2 wherein the isolation of CD10⁻ spermatozoid is carried out by magnetic-activated cell sorting.

4. The method according to any of claims 1, 2 or 3 wherein the starting spermatozoid population is isolated from a subject which is infertile, normozoospermic, astenozoospermic, teratozoospermic, oligozoospermic or any combination thereof.

5. The method according to any of claims 1, 2, 3 or 4 wherein the starting spermatozoid cell population is of a mammal.

6. The method according to claim 5 wherein the mammal is a human, porcine, bovine, equine, canine, murine or feline.

7. Use of a CD10-binding agent for the preparation of a spermatozoid cell population enriched in CD10⁻ spermatozoids.

8. Use of a CD10-binding agent for the preparation of a spermatozoid cell population having a high degree of DNA integrity.

9. Use of a CD10-binding for the preparation of a spermatozoid cell population having a low level of aneuploidies.

10. The use according to any of claims 7, 8 or 9 wherein the CD10-binding agent is an anti-CD10 antibody.

11. A method for determining the fitness of a human sperm population for fertilization which comprises determining the content of CD10⁻ spermatozoids in a said population, wherein increased levels of CD10⁻ spermatozoids in the population with respect to a reference value are indicative that the human sperm is adequate for fertilization.

12. A method for determining the DNA integrity of a human sperm population which comprises determining the content of CD10⁻ spermatozoids in a said population, wherein increased levels of CD10⁻ spermatozoids in the population with respect to a reference value are indicative that the sperm population shows a high degree of DNA integrity.

13. A method for determining the number of aneuplodies in a human sperm population which comprises determining the content of CD10⁻ spermatozoids in a said population, wherein increased levels of CD10⁻ spermatozoids in the population with respect to a reference value are indicative that the sperm population shows a low level of aneuploidies.

14. The method according to any of claims 11, 12 or 13, wherein the content of CD10⁻ spermatozoids is determined with a CD10-binding agent.

15. The method according to claim 14 wherein the CD10-binding agent is an antibody.

16. The method according to any of claims 11 to 15 wherein the human sperm is isolated from a subject which is infertile, normozoospermic, asthenozoospermic, teratozoospermic, oligozoospermic or any combination thereof.

17. The method according to any of claims 11 to 16 wherein the human sperm population is a semen sample.

## Patentansprüche

1. Eine Methode zur Gewinnung einer Spermatozoid-Zellpopulation, die mit CD10⁻ Spermatozoiden angereichert ist, aus einer Ausgangsspermienpopulation, umfassend:
(i) Inkontaktbringen der Ausgangsspermienpopulation mit einem CD10-Bindemittel und
(ii) Isolieren von Spermien aus der Ausgangspopulation, die nicht an das CD10-Bindemittel binden.

2. Die Methode nach Anspruch 1, wobei das CD10-Bindemittel ein Anti-CD10-Antikörper ist.

3. Die Methode nach Anspruch 2, wobei die Isolierung der CD10⁻Spermatozoiden durch magnetisch aktivierte Zellsortierung durchgeführt wird.

4. Die Methode nach einem der Ansprüche 1, 2 oder 3, wobei die Ausgangsspermienpopulation aus einem Subjekt isoliert wird, das unfruchtbar, normozoospermisch, astenozoospermisch, teratozoospermisch, oligozoospermisch oder eine Kombination davon ist.

5. Die Methode nach einem der Ansprüche 1, 2, 3 oder 4, wobei die Ausgangsspermienpopulation aus einem Säugetier ist.

6. Die Methode nach Anspruch 5, wobei das Säugetier ein Mensch, ein Schwein, ein Rind, ein Pferd, ein Hund, eine Maus oder eine Katze ist.

7. Verwendung eines CD10-Bindemittels zur Herstellung einer Spermatozoid-Zellpopulation, die mit CD10⁻ Spermatozoiden angereichert ist.

8. Verwendung eines CD10-Bindungsmittels zur Herstellung einer Spermatozoid-Zellpopulation mit hoher DNA-Integrität.

9. Verwendung einer CD10-Bindung zur Herstellung einer Spermatozoid-Zellpopulation mit einem niedrigen Aneuploidiegrad.

10. Die Verwendung nach einem der Ansprüche 7, 8 oder 9, wobei das CD10-Bindungsmittel ein Anti-CD10-Antikörper ist.

11. Eine Methode zur Bestimmung der Eignung einer menschlichen Spermienpopulation für die Befruchtung, die darin besteht, den Gehalt an CD10⁻ Spermatozoiden in der genannten Population zu bestimmen, wobei erhöhte Werte an CD10⁻ Spermatozoiden in der Population in Bezug auf einen Referenzwert darauf hindeuten, dass das menschliche Spermium für die Befruchtung ausreichend ist.

12. Eine Methode zur Bestimmung der DNA-Integrität einer menschlichen Spermienpopulation, die darin besteht, den Gehalt an CD10⁻ Spermatozoiden in der genannten Population zu bestimmen, wobei erhöhte Werte an CD10⁻ Spermatozoiden in der Population in Bezug auf einen Referenzwert darauf hindeuten, dass die Spermienpopulation eine hohe DNA-Integrität aufweist.

13. Eine Methode zur Bestimmung der Anzahl der Aneuplodien in einer menschlichen Spermienpopulation, die die Bestimmung des Gehalts von CD10⁻ Spermatozoiden in einer genannten Population umfasst, wobei erhöhte Werte an CD10- Spermatozoiden in der Population in Bezug auf einen Referenzwert darauf hindeuten, dass die Spermienpopulation ein niedriges Niveau von Aneuploiden aufweist.

14. Die Methode nach einem der Ansprüche 11, 12 oder 13, wobei der Gehalt an CD10⁻ Spermatozoiden mit einem CD10-Bindemittel bestimmt wird.

15. Die Methode nach Anspruch 14, wobei das CD10-Bindemittel ein Antikörper ist.

16. Die Methode nach einem der Ansprüche 11 bis 15, wobei das menschliche Sperma aus einem Subjekt isoliert wird, das unfruchtbar, normozoospermisch, astenozoospermisch, teratozoospermisch, oligozoospermisch oder eine Kombination davon ist.

17. Die Methode nach einem der Ansprüche 11 bis 16, wobei die menschliche Spermienpopulation eine Samenprobe ist.

## Revendications

1. Procédé d'obtention d'une population de cellules de spermatozoïdes enrichie en spermatozoïdes CD10⁻ à partir d'une population de départ de spermatozoïdes, comprenant :
(i) la mise en contact de la population de départ de spermatozoïdes avec un agent de liaison au CD10 et
(ii) l'isolation des spermatozoïdes de la population de départ qui ne se lient pas à l'agent de liaison au CD10.

2. Procédé selon la revendication 1, dans lequel l'agent de liaison au CD10 est un anticorps anti-CD10.

3. Procédé selon la revendication 2, dans lequel l'isolation des spermatozoïdes CD10⁻ est effectuée par tri cellulaire activé magnétiquement.

4. Procédé selon l'une quelconque des revendications 1, 2 ou 3, dans lequel la population de départ de spermatozoïdes est isolée d'un sujet qui est infertile, normozoospermique, asthénozoospermique, tératozoospermique, oligozoospermique ou toute combinaison de ceux-ci.

5. Procédé selon l'une quelconque des revendications 1, 2, 3 ou 4, dans lequel la population de départ de cellules de spermatozoïdes provient d'un mammifère.

6. Procédé selon la revendication 5, dans lequel le mammifère est un être humain, un porcin, bovin, équin, canin, murin ou félin.

7. Utilisation d'un agent de liaison au CD10 pour la préparation d'une population de cellules de spermatozoïdes enrichie en spermatozoïdes CD10⁻.

8. Utilisation d'un agent de liaison au CD10 pour la préparation d'une population de cellules de spermatozoïdes ayant un haut degré d'intégrité de l'ADN.

9. Utilisation d'un agent de liaison au CD10 pour la préparation d'une population de cellules de spermatozoïdes présentant un faible niveau d'aneuploïdies.

10. Utilisation selon l'une quelconque des revendications 7, 8 ou 9, dans laquelle l'agent de liaison au CD10 est un anticorps anti-CD10.

11. Procédé pour déterminer l'aptitude à la fécondation d'une population spermatique humaine, qui comprend la détermination de la teneur en spermatozoïdes CD10⁻ dans ladite population, dans lequel des niveaux accrus de spermatozoïdes CD10⁻ dans la population par rapport à une valeur de référence sont indicatifs du fait que la population spermatique est apte à la fécondation.

12. Procédé pour déterminer l'intégrité de l'ADN d'une population spermatique humaine, qui comprend la détermination de la teneur en spermatozoïdes CD10⁻ dans ladite population, dans lequel des niveaux accrus de spermatozoïdes CD10⁻ dans la population par rapport à une valeur de référence sont indicatifs du fait que la population spermatique présente un haut degré d'intégrité de l'ADN.

13. Procédé pour déterminer le nombre d'aneuploïdies dans une population spermatique humaine, qui comprend la détermination de la teneur en spermatozoïdes CD10⁻ dans ladite population, dans lequel des niveaux accrus de spermatozoïdes CD10⁻ dans la population par rapport à une valeur de référence sont indicatifs du fait que la population spermatique présente un faible niveau d'aneuploïdies.

14. Procédé selon l'une quelconque des revendications 11, 12 ou 13, dans lequel la teneur en spermatozoïdes CD10⁻ est déterminée avec un agent de liaison au CD10.

15. Procédé selon la revendication 14, dans lequel l'agent de liaison au CD10 est un anticorps.

16. Procédé selon l'une quelconque des revendications 11 à 15, dans lequel le sperme humain est isolé d'un sujet qui est infertile, normozoospermique, asthénozoospermique, tératozoospermique, oligozoospermique ou toute combinaison de ceux-ci.

17. Procédé selon l'une quelconque des revendications 11 à 16, dans lequel la population spermatique humaine est un échantillon de sperme.
